# EUROPEAN PATENT SPECIFICATION

(11) **EP 1 220 830 B1**
(45) Date of publication and mention of the grant of the patent: **02.05.2007**
(21) Application number: 00965396.5
(22) Date of filing: 24.09.2000
(51) Int. Cl.: A61K 38/48, A61P 9/14

(54) **USE OF ELASTASE FOR OPENING OBSTRUCTED ARTERIES AND VEINS**
VERWENDUNG VON ELASTASE ZUR ÖFFNUNG VERSTOPFTER ARTERIEN UND VENEN
UTILISATION DE L'ELASTASE POUR OUVRIR DES ARTERES ET VEINES OBSTRUEES

(30) Priority: 24.09.1999 US 155938 P
(43) Date of publication of application: 10.07.2002
(73) Proprietor: Proteon Therapeutics, Inc., Kansas City, MO 64111 (US)
(72) Inventor: FRANANO, Nicholas, F., Laurel, MD 20723 (US)
(74) Representative: Adam, Holger
(86) International application number: PCT/US2000/026237
(87) International publication number: WO 2001/021574

(56) References cited:
- WO-A1-96/18725
- US-A- 4 803 294
- US-A- 5 116 615
- US-A- 5 397 694
- US-A- 5 489 261
- US-A- 5 922 322
- US-A- 6 143 744

## Description

This application claims the benefit of U. S. Provisional Application Serial No. 60/155938 filed September 24,1999, which is incorporated by reference herein in its entirety.

### STATEMENT REGARDING GOVERNMENT RIGHTS

The U. S.Government has certain rights in this invention.

### BACKGROUND OF THE INVENTION

### 1. Field of the Invention.

The present invention relates to the use of elastases for enlarging the diameter of an artery or vein in a human subject in need thereof. In particular, the present invention relates to the use of an elastase for the manufacture of a medicament for local administration to the wall of an artery or vein to enlarge the diameter of the artery or vein in a human subject in need thereof.

### 2. Background.

Obstructions to biological conduits frequently result from trauma to the conduit which can result from transplant, graft or other surgical procedures wherein the extracellular matrix of the obstructing tissue largely comprises collagen. Balloon angioplasty is a common initial treatment for stenosis or stricture obstruction that yields excellent initial results (Pauletto, Clinical Science, (1994) 87: 467-79). However, this dilation method does not remove the obstructing tissue. It only stretches open the lumen, the trauma of which has been associated with the release of several potent cytokines and growth factors that can cause an injury which induces another round of cell proliferation, cell migration toward the lumen and synthesis of more extracellular matrix. Consequently, balloon angioplasty is associated with restenosis in nearly all patients (Pauletto, Clinical Science, (1994) 87: 467-79). There is currently no treatment that can sustain patency over the long term.

The extracellular matrix, which holds a tissue together, is composed primarily of collagen, the major fibrous component of animal extracellular connective tissue (Krane, J. Investigative Dermatology (1982) 79: 83s-86s; Shingleton,Biochem. Cell Biol., (1996) 74: 759-75). The collagen molecule has a base unit of three strands, of repeating amino acids coiled into a triple helix. These triple helix coils are then woven into a right-handed cable. As the collagen matures, cross-links form between the chains and the collagen becomes progressively more insoluble and resistant to lysis. When properly formed, collagen has a greater tensile strength than steel. Not surprisingly, when the body builds new tissue collagen provides the extracellular structural framework such that the deposition of hard collagen in the lesion can result in duct obstruction.

Benign biliary stricture results in obstruction of the flow of bile from the liver can result in jaundice and hepatic dysfunction. If untreated, biliary obstruction can result in hepatic failure and death. Billary strictures can form after duct injury during cholecystectomy. They can also form at biliary anastomoses after liver transplantation and other biliary reconstructive surgeries (Vitale,Am. J. Surgery (1996) 171: 553-7; Lilliemoe, Annals of Surgery (1997) 225).

Historically, benign biliary stricture has been treated surgically by removing the diseased duct segment and reconnecting the duct end-to-end, or connecting the duct to the bowel via a hepaticojejunostomy loop (Lilliemoe, Annalsof Surgery (1997) 225). These long and difficult surgeries have significant morbidity and mortality due to bleeding, infection, biliary leak, and recurrent biliary obstruction at the anastomosis. Post-operative recovery takes weeks to months. More recently, minimally invasive treatments such as percutaneous balloon dilation have been utilized, yielding good initial biliary patency surgeries (Vitale,Am. J. Surgery (1996) 171: 553-7; Lilliemoe,Annals of Surgery (1997) 225). However, balloon dilation causes a localized injury, inducing a healing response that often results in restenosis (Pauletto, Clinical Science, (1994) 87: 467-79).Long-term stenting at the common bile duct with flexible biliary drainage catheters is another minimally invasive alternative to surgery (Vitale,Am. J. Surgery (1996) 171:553-7). However, these indwelling biliary drainage catheters often become infected, or clogged with debris, and must be changed frequently. At present, long-term treatment of biliary stricture remains a difficult clinical problem.

Patients with chronic, end-stage renal failure may require replacement of their kidney function in order to survive. In the United States, long-term hemodialysis is the most common treatment method for end stage chronic renal failure in the U. S. In 1993, more-than 130,000 patients underwent long term hemodialysis(Gaylord, J.Vascular and Interventional Radiology (1993) 4: 103-7), More than 80% of these patients implement hemodialysis through the use of a synthetic arteriovenous graft (Windus,Am. J. Kidney Diseases (1993) 21: 457-71). In a majority of these patients, the graft consists of a 6 mm Gore-Tex tube that is surgically implanted between an artery and a vein, usually in the forearm or upper arm. This high flow conduit can then be accessed with needles for hemodialysis sessions.

Nearly all hemodialysis grafts fail, usually within two years, and a new graft must be created surgically to maintain hemodialysis. These patients face repeated interruption of hemodialysis, and multiple hospitalizations for radiological and surgical procedures. Since each surgical graft revision consumes more available vein, eventually they are at risk for mortality from lack of sites for hemodialysis access. One estimate placed the cost of graft placement, hemodialysis, treatment of complications, placement of venous catheters, hospitalization costs, and time away from work at as much as $500 million, in 1990 alone (Windus,Am. J. Kidney Diseases (1993) 21: 457-71).

The most frequent cause of hemodialysis graft failure is thrombosis, which is often due to development of a stenosis in the vein just downstream from the graftvein anastomosis (Safa, Radiology (1996) 199: 653-7. Histologic analysis of the stenosis reveals a firm, pale, relatively homogeneous lesion interposed between the intimal and medial layers of the vein which thickens the vessel wall and narrows the lumen (Swedberg, Circulation (1989) 80: 1726-36). This lesion, which has been given the name intimal hyperplasia is composed of vascular smooth muscle cells surrounded by an extensive extracellular collagen matrix (Swedberg, Circulation (1989) 80: 1726-36; Trerotola, J.Vascular and Interventional Radiology (1995) 6: 387-96). Balloon angioplasty is the most common initial treatment for stenosis of hemodialysis grafts and yields excellent initial patency results (Safa, Radiology (1996) 199: 653-7). However, this purely mechanical method of stretching open the stenosis causes an injury which induces another round of cell proliferation, cell migration toward the lumen and synthesis of more extracellular matrix. Consequently, balloon angioplasty is associated with restenosis in nearly all patients (Safa, Radiology (1996) 199: 653-7). There is currently no treatment which can sustain the patency of synthetic arteriovenous hemodialysis grafts over the long term.

Intimal hyperplasia research has focused largely on the cellular component of the lesion. The use of radiation and pharmaceutical agents to inhibit cell proliferation and migration are active areas of research (Hirai,ACTA Radiologica (1996) 37: 229-33; Reimers,J. Invasive Cardiology (1998) 10: 323-31; Choi,J Vascular Surgery (1994) 19: 125-34). To date, the results of these studies have been equivocal, and none of these new treatments has gained wide clinical acceptance. This matrix is composed predominantly of collagen and previous work in animals has demonstrated that systemic inhibition of collagen synthesis decreases the production of intimal hyperplasia (Choi, Archives of Surgery (1995) 130: 257-261).

During normal tissue growth and remodeling, existing collagen matrices must be removed or modified. This collagen remodeling is carried out by macrophages and fibroblasts, two cell types which secrete a distinct class of proteases called"collagenases" (Swedberg, Circulation (1989) 80: 1726-36; Trerotola, J. Vascular and Interventional Radiology (1995) 6: 387-96; Hirai, ACTA Radiologica (1996) 37: 229-33). These collagenases rapidly degrade insoluble collagen fibrils to small, soluble peptide fragments, which are carried away from the site by the flow of blood and lymph.

See also U. S. Patents 5,981,568; 5,409,926; and 6,074,659.

Moreover, US-A 5,922,322 discloses a method of degrading fibrin(ogen), comprising:
contacting said fibrin(ogen) with an amount of fibrinolytic matrix metalloproteinase effective for cleaving the fibrin(ogen), wherein said fibrinolytic matrix metalloproteinase cleaves the fibrin(ogen) at the γGly404-Ala405 peptide bond.

US-A 5,116,615 teaches a method of treating prostatic hypertrophy in a living mammal comprising direct intraprostatic injection of a composition comprising a therapeutically effective concentration of collagenase and at least one enzyme selected from the group consisting of hyaluronidase, elastase, trypsin, chymotrypsin, pronase, DNase I, bromelin, clostripain, thermolysin, neuraminidase, phospholipase, cholesterol esterase, dispase, subtilisin, papain, chymopapain, plasminogen activator, plasmin, streptokinase, urokinase, fibrinolysin, serrathiopeptidase, pancreatin, amylase lysozyme, cathepsin-G, and the PMN leukocyte serine proteases; wherein said composition causes the dissolution and regression of hypertrophied prostatic tissue thereby providing relief from the obstructive symptoms associated with prostatic hypertrophy.

It thus would be desirable to provide new methods to relieve obstructions blocking flow through biolgical conduits.

### SUMMARY OF THE INVENTION

The present invention is based on the finding of new methods and systems for relieving an obstruction in a biological conduit, e.g. mammalian vasculature. Said methods of the invention include administration to an obstruction site of a therapeutic agent that can preferably degrade (in vivo) the extracellular matrix of the obstructing tissue, particularly collagen and/or elastin. Preferred methods include administration to an obstruction of an enzyme or a mixture of enzymes that are capable of degrading key extracellular matrix components (including collagen and/or elastin) resulting in the solubilization or other removal of the obstructing tissue.

Said methods and systems can be applied to a variety of specific therapies. For example, said methods include treatment of bilary stricture with the use of exogenous collagenase, elastase or other agent, whereby an enzyme composition comprising collagenase, elastase or other agent is directly administered to or into (such as by catheter injection) the wall of the lesion or other obstruction. The enyzme (s) dissolves the collagen and/or elastin in the extracellular matrix, resulting in the solubilization of fibrous tissue from the duct wall near the lumen, and a return of duct flow or opening.

Preferably, the therapeutic agent is delivered proxumately to a targeted site, e.g. by injection, catheter delievery or the like.

The present invention provides for the use of an elastase for the manufacture of a medicament for local administration to the wall of an artery or vein to enlarge the diameter of the artery or vein in a human subject in need thereof.

The present invention provides for the use of an elastase for the manufacture of a medicament for local administration to the wall of an artery or vein to enlarge the diameter of the artery or vein in a human subject in need thereof, wherein the artery or vein is connected to an arteriovenous hemodialysis graft in the human subject.

The present invention provides for the use of an elastase for the manufacture of a medicament for local administration to the wall of an artery or vein to enlarge the diameter of the artery or vein in a human subject in need thereof, wherein the medicament is for local administration to the wall of a vein for use in hemodialysis. In certain embodiments, the medicament is for administration to the wall of the vein for use in forming an arteriovenous graft.

The present invention provides for the use of an elastase for the manufacture of a medicament for local administration to the wall of an artery or vein to enlarge the diameter of the artery or vein in a human subject in need thereof, wherein the artery or vein is obstructed, for example by intimal hyperplasia or by stenosis (for example wherein the stenosis permits passage of an insufficient volume of blood prior to the treatment).

In certain aspects of any of the foregoing embodiments, the subject is suffering from coronary obstruction.

In certain aspects of any of the foregoing embodiments, the medicament is administered by a catheter. In specific aspects of any of the foregoing embodiments, the medicament is administered directly into the wall of the artery or vein.

In a preferred aspect of the any of the foregoing embodiments, wherein the medicament comprises a pancreatic elastase.

In another preferred aspect of the any of the foregoing embodiments, the medicament does not comprise a collagenase.

In a specific aspect of any the preceding embodiments, the medicament causes enlargement of the diameter of the artery or vein by proteolysis of elastin in the wall of the artery or vein from the outside in.

Other aspects of the invention are disclosed infra.

### BRIEF DESCRIPTION OF THE DRAWING

FIG. I shows a common bile duct in a dog with a high grade stricture;
FIG. 2 shows a common bile duct in a dog with a high grade stricture after treatment;
FIG. 3 is a histology picture of a normal common bile duct from a dog; and
FIG. 4 is a histology picture of a common bile duct stricture from a dog with a high grade stricture before treatment.

### DETAILED DESCRIPTION OF THE INVENTION

The invention provides for introduction of a therapeutic agent that is capable of degrading an extracellular matrix components to thereby facilitate the reopening of a constricted biological conduit. In particular, the invention provides for introduction to an obstructed bioliogical conduit of a therapeutic agent that degrades collagen and/or elastin. The use of the present invention further provides methods of dialating a biological conduit by introducing a therapeutic agent comprising an elastase into a biological conduit, preferably an isolated segment of the conduit.

In one embodiment of the present invention, the degradation of a stricture, lesion or other obstruction is accomplished by introducing one or more therapeutic agents that are capable of degrading one or more extracellular matrix components thereby facilitating the reopening of the constricted segment of the conduit. Major structural components of the extracellular matrix include collagen and elastin.

Preferred therapeutic agents for use in accordance with the invention are able to interact with and degrade either one or both of collagen and elastin.

As discussed above, a variety of compositions may be used in the methods and systems of the invention. Preferred therapeutic compositions comprise one or more agents that can solubilize or otherwise degrade collagen or elastin in vivo.

Appropriate therapeutic agents can comprise at least one and frequently several enzymes such that the therapeutic agent is capable of degrading both significant matrix components of tissue obstruction. A particularly preferable therapeutic agent will comprise an elastase, particularly pancreatic elastase, an enzyme capable of degrading elastin.

In a further aspect of the present invention, the methods further include means to prevent damage to tissue that is not associated with conduit obstruction.

Fragments of therapeutic agents also can be administered to a patient in accordance with the invention. For example, fragments of the above-mentioned elastases can be administered to a patient provided such fragments provide the desired therapeutic effect, i.e. degradation of obstruction of a biological conduit. As referred to herein, an elastase includes therapeutically effective elastase fragments.

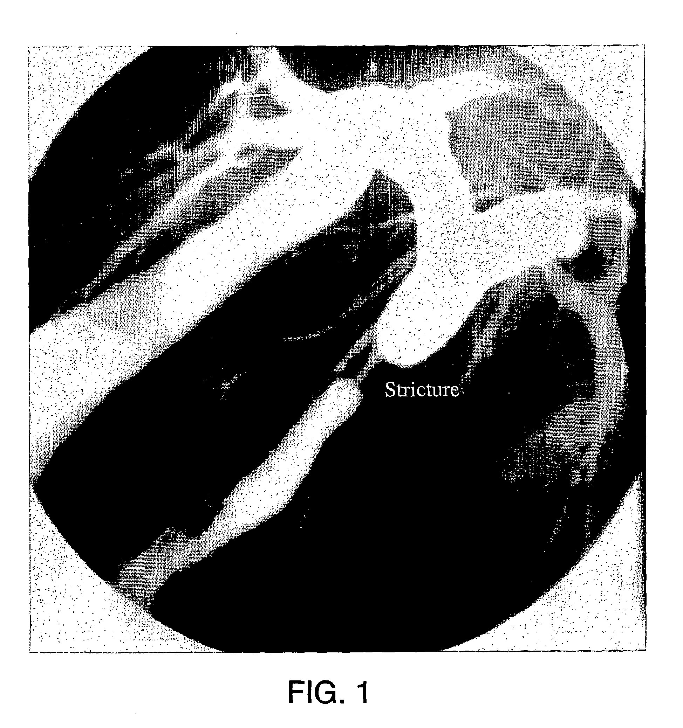

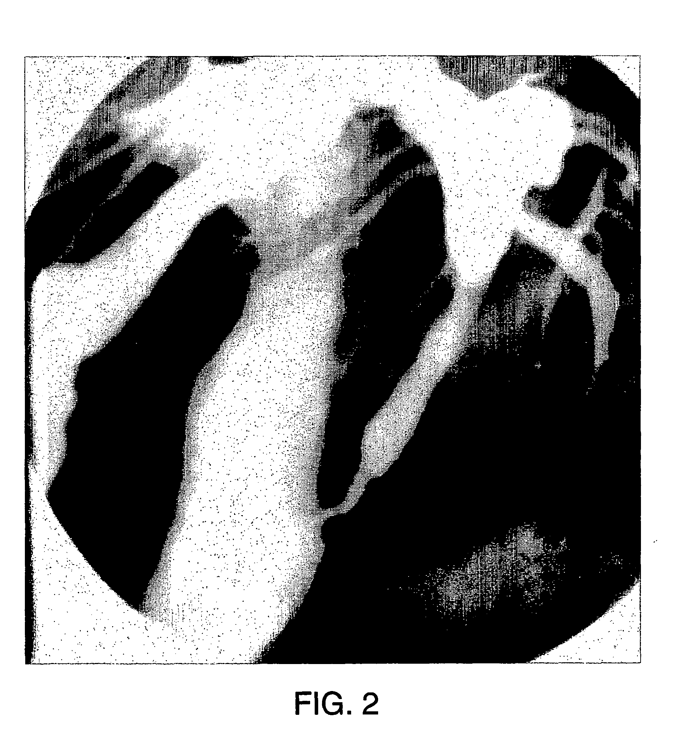

In a preferred aspect of the invention, a therapeutic agent comprising at least one enzyme capable of degrading elastin, collagen or both is delivered to the targeted obstruction site with a catheter. Preferred catheters are capable of directly localizing a therapeutic agent directly into the extracellular matrix of the obstruction. Particularly preferable catheters are able of delivering accurate doses of therapeutic agent with an even distribution over the entire obstructed area of the conduit. One particularly preferred example of a catheter for use in the method of the present invention is the Infiltrator® catheter produced by InterVentional Technologies Corporation (IVT) (San Diego, CA), which delivers a precisely controlled dosage of a drug directly into a selected segment of vessel wall (Figure 1)(Reimers, J. Invasive Cardiology (1998) 10:323-331; Barath, Catherterization and Cardiovascular Diagnosis (1997) 41: 333-41; Woessner, Biochem. Cell Biol. (1996) 74: 777-84). Using this preferred catheter a therapeutic agent can be delivered at low pressure via a series of miniaturized injector ports mounted on the balloon surface. When the positioning balloon is inflated, the injector ports extend and enter the vessel wall over the 360 surface of a 15 mm segment of vessel. Each injector port is less than 0.0035 inch in size. Drug delivery can be performed in less than 10 seconds, with microliter precision and minimal immediate drug washout. The injected drug is delivered homogeneously in the wall of the vessel or duct (Figure 2). The triple lumen design provides independent channels for guidewire advancement, balloon inflation and drug delivery. Trauma associated with injector port penetration is minimal and the long-term histologic effects are negligible (Woessner, Biochem. Cell Biol. (1996) 74: 777-84). In addition, the device has been engineered such that the injector ports are recessed while maneuvering in the vessel. Additionally, the Infiltrator® catheter is capable of balloon inflation with sufficient force for angioplasty applications. The excellent control of drug delivery observed with Infiltrator® can be significant since preferred therapeutic agents of the present invention potentially can degrade collagen and/or elastin in nearly all forms of tissue in a non-specific manner.

In yet another embodiment of the present invention, a therapeutic dose is employed which will restore conduit flow while maintaining conduit wall integrity. Several parameters need to be defined to maximize method efficiency, including the amount of enzyme to be delivered, the volume of enzyme solution to be injected so that the reopening of the conduit occurs with a single dose protocol. Ideally repeat or multiple dosing is reserved only for patients who have an incomplete response to the initial injection.

In regards to the volume of therapeutic agent solution delivered, preferably the conduit wall is not saturated completely, as this can lead to transmural digestion and conduit rupture. Instead, the optimal dose is determined by targeting the thickness of the wall (from the outside in) which needs to be removed in order to restore adequate flow, while leaving the remaining wall intact. An overly dilute solution will be ineffective at collagen lysis while an overly concentrated solution will have a higher diffusion gradient into the surrounding tissues, thereby increasing the risk of transmural digestion and rupture.

It will be appreciated that actual preferred dosage amounts of therapeutic agents in a given therapy will vary according to e.g. the specific compound being utilized, the particular composition formulated, the mode of administration and characteristics of the subject, e.g. the species, sex, weight, general health and age of the subject. Optimal administration doses for a given protocol of administration can be readily ascertained by those skilled in the art using dosage determination tests, including those described above and in the examples which follow.

Therapeutic agents of the invention are suitably administered as a pharmaceutical composition with one or more suitable carriers. Therapeutic agents of the invention are typically formulated in injectable form, e.g. with the therapeutic agent dissolved in a suitable fluid carrier. See the examples which follow for preferred compositions.

As discussed above, the methods and systems of the invention can be employed to treat (including prophylactic treatment) a variety of diseases and disorders. In particular, methods and systems of the invention can be employed to relieve or otherwise treat a variety of lesions and other obstructions found in common bile ducts or vascular systems. Methods of the invention are also useful to relieve lesions and other obstructions in other biological conduits including e.g. ureterer, pancreatic duct, bronchi, coronary and the like.

The invention also includes prophalytic-type treatment, e.g. methods to dialate a biological conduit whereby the increased conduit diameter obviates the potential of obstruction formation within a conduit. Temporary and partial degredation of the elastin component of a conduit wall reduces the elasticity of the conduit thereby facilitating modifications of the size and shape of the conduit.

Introducing a dose of therapeutic agent in accordance with the invention into the lumen of an isolated conduit or some section thereof results in complete or partial diffusion of the therapeutic agent into the wall of the isolated conduit during a specified period of time. Subsequent pressurization of the treated region either while the region is still isolated or after removing the means of isolation increases the lumen diameter by dilation. Regeneration of the conduit elastin framework results in a conduit with a larger lumen diameter and without compromising the structural integrit graft. Frequently the lumenal diameter of the venous outflow is smaller than the graft lumenal diameter. Development of a stenosis due to intimal hyperplasia can further reduce the lumenal diameter of the venous outflow such that an insufficient volume of blood passes through the venous outflow. To prevent intimal hyperplasia and stenosis formation, dilating the venous outflow vein using the above described method of partially degrading the elastin component of the vascular wall downstream of the site of graft implantation such that the lumenal diameter of the venous outflow is similar to or larger than the diameter of the interposed loop graft reduces the likelihood of forming of a stenosis due to intimal hyperplasia. Venous dialation can be preformed either before or after interposing a graft between the artery and vein.

All documents mentioned herein are incorporated herein by reference. The present invention is further illustrated by the following non-limiting examples.

### Example 1: Tissue digestion analysis.

The protocol of the following example is a detailed description of a "standard in vitro tissue digestion assay"as referred to herein.

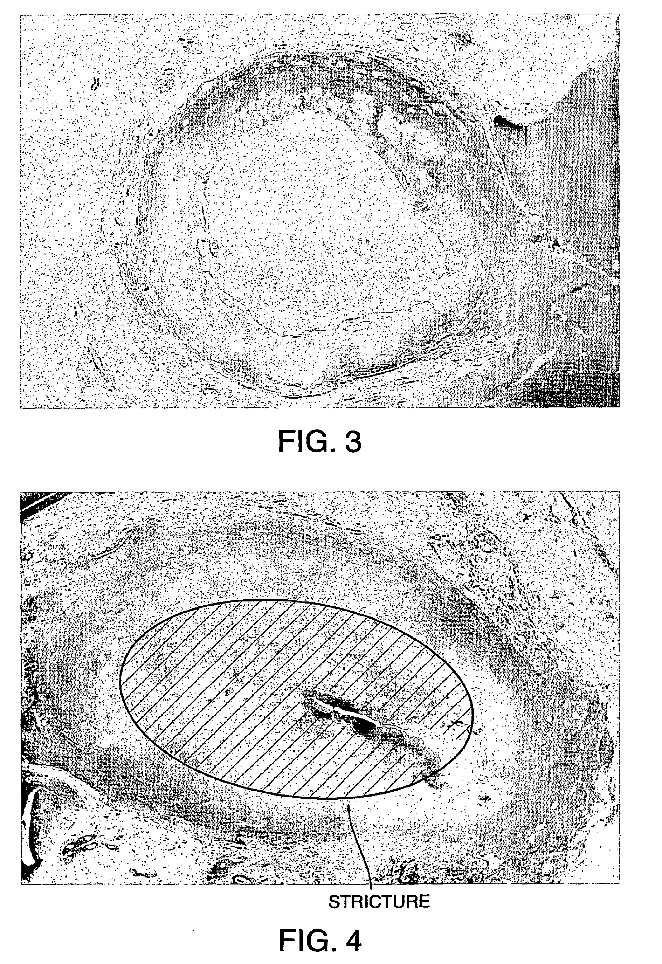

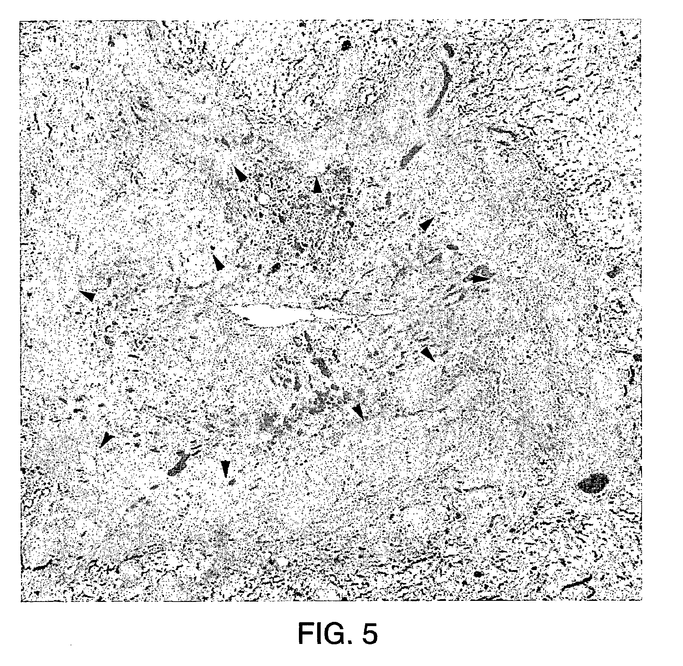

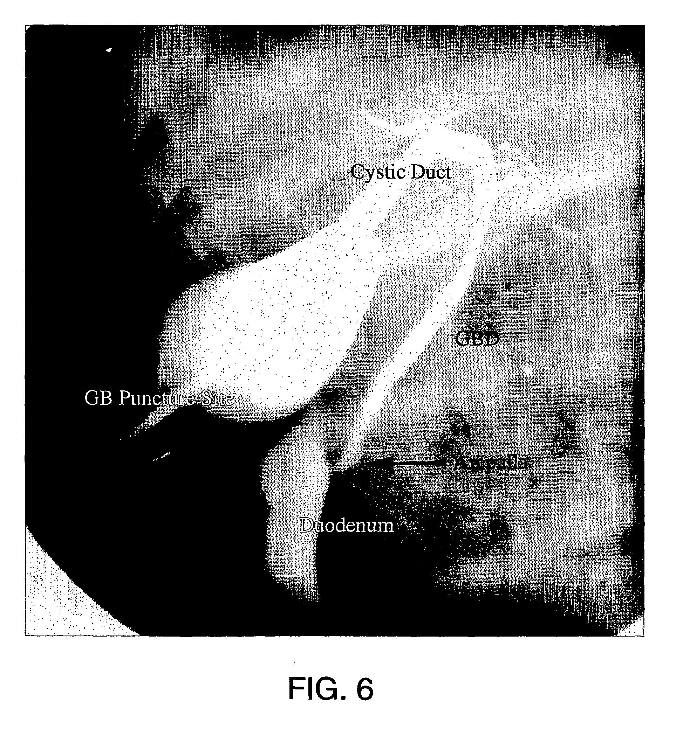

The rate of tissue digestion, which is composed mostly of collagen, by a mixture of collagenase and elastase, proteolytic enzymes with activity respectfully against collagen and elastin, was determined. Trypsin inhibitor was added to negate the affect of any residual trypsin activity. Briefly, fresh pig tendon was excised, trimmed, washed, blotted dry and weighed. Individual tendon pieces were suspended in 3.58mg/ml HEPES buffer at neutral pH and various concentrations of enzymes were added. Iodinated radiographic contrast was added in various concentrations to some of the enzyme solutions. The tissue digestion was carried out in a water bath at 37°C. At various time points, the tendon pieces were removed from the enzyme solution, washed, blotted dry and weighed. Each time point was derived from the average of three samples. The effect of enzyme concentration on tissue digestion rates was studied. As expected, increasing the concentration of enzymes in vitro increased the rate of tissue digestion (Figure 3). Buffer alone had no effect on the tissue. Extrapolating digestion rates in vitro to an in vivo situation has proven difficult. For Dupuytren's contractures, the effective dose for transecting fibrous cords in vitro was 500 ABC. However, the effective in vivo dose was 10,000 ABC units.

The effect of iodinated radiographic contrast material on tissue digestion rates was also studied (Figure 4). This study was performed to monitor enzyme delivery by mixing it with contrast prior to injection. These results demonstrate that Omnipaque 350 iodinated contrast material inhibits enzyme activity at radiographically visible (35%) concentrations, but not at lower (1-5%) concentrations (Figure 4). Similar results were observed with Hypaque 60 contrast.

### Example 2. Determining dose dependant in vitro activity of a therapeutic agent including collagenase, elastase, and a trypsin inhibitor.

The effect of enzyme concentration on tissue digestion rates was studied (Figure 3). The"lx"tissue sample was treated with collagenase 156 Mandel units/ml + elastase 0.125 mg/ml + trypsin inhibitor038 mg/mg, The"2x"sample was treated with collagenase 312 Mandel units/ml + elastase 0.25 mg/ml + trypsin inhibitor 0.76 mg/ml.The"5x"sample was treated with collagenase 780 Mandel units/ml + elastase 0.625 mg/ml + trypsin inhibitor 1.9 mg/ml. All digestion volumes were 0.5 ml. Increasing the concentration of enzymes in vitro increased the rate of tissue digestion (Figure 3). Buffer alone had no effect on the tissue. An effective in vivo dose was found to be 10,000 ABC units.

### Example 3. Determining the effect of iodinated radiographic contrast material on tissue digestion rates facilitate monitoring enzyme delivery prior to injection of a therapeutic agent comprising a contrast material into a patient.

The"35% Omnipaque"tissue sample was treated with collagenase 156 Mandel units/ml + elastase 0.125 mg/ml + 0.38 trypsin inhibitor with 35% OmniPaque 350 contrast (volume: volume). The"5% Omnipaque"sample was treated with collagenase 312 Mandel units/ml + elastase 0.25 mg/ml +0.76 trypsin inhibitor with 5% Omnipaque 350 (volume: volume).The"1% Omnipaque"sample was treated with collagenase 312 Mandel units/ml + elastase 0:25 mg/ml + 0.76 trypsin inhibitor with1 % Omnipaque 350. All digestion volumes were 0.5 ml. These results demonstrate that Omnipaque 350 iodinated contrast material inhibits enzyme activity at radiographically visible (35%) concentrations, but not at lower (1-5%) concentrations (Figure 4). Similar results were observed with Hypaque 60 contrast.

The invention has been described in detail with reference to preferred embodiments thereof. However, it will be appreciated that those skilled in the art, upon consideration of this disclosure, may make modifications and improvements within the spirit and scope of the invention as set forth in the following claims.

## Claims

1. Use of an elastase for the manufacture of a medicament for local administration to the wall of an artery or vein to enlarge the diameter of the artery or vein in a human subject in need thereof.

2. The use according to claim 1, wherein the artery or vein is connected to an arteriovenous hemodialysis graft in the human subject.

3. The use according to claim 1, wherein the medicament is for local administration to the wall of a vein for use in hemodialysis.

4. The use according to claim 3, wherein the medicament is for administration to the wall of the vein for use in forming an arteriovenous graft.

5. The use according to any of the preceding claims, wherein the artery or vein is obstructed.

6. The use according to claim 5, wherein the artery or vein is obstructed by intimal hyperplasia.

7. The use according to claim 5, wherein the artery or vein is obstructed by stenosis .

8. The use according to claim 7, wherein the stenosis permits passage of an insufficient volume of blood prior to the treatment.

9. The use according to any of the preceding claims, wherein the subject is suffering from coronary obstruction.

10. The use according to any of the preceding claims, wherein the medicament is administered by a catheter.

11. The use according to any of the preceding claims, wherein the medicament is administered directly into the wall of the artery or vein.

12. The use according to any of the preceding claims, wherein the medicament comprises a pancreatic elastase.

13. The use according to any of the preceding claims, wherein the medicament does not comprise a collagenase.

14. The use according to any of the preceding claims, wherein the medicament causes enlargement of the diameter of the artery or vein by proteolysis of elastin in the wall of the artery or vein from the outside in.

## Patentansprüche

1. Verwendung einer Elastase zur Herstellung eines Medikaments für eine lokale Verabreichung an die Wand einer Arterie oder Vene, um den Durchmesser der Arterie oder Vene bei einem Menschen, der Bedarf dafür hat, zu vergrößern.

2. Verwendung gemäß Anspruch 1, wobei die Arterie oder Vene mit einem arteriovenösen Hämodialyse-Transplantat ("arteriovenous hemodialysis graft") beim Menschen verbunden ist.

3. Verwendung gemäß Anspruch 1, wobei das Medikament für eine lokale Verabreichung an die Wand einer Vene für eine Verwendung in Hämodialyse ist.

4. Verwendung gemäß Anspruch 3, wobei das Medikament für eine Verabreichung an die Wand der Vene für eine Verwendung zur Bildung eines arteriovenösen Transplantats ist.

5. Verwendung gemäß einem der vorangehenden Ansprüche, wobei die.Arterie oder Vene verstopft ist.

6. Verwendung gemäß Anspruch 5, wobei die Arterie oder Vene durch Intimahyperplasie verstopft ist.

7. Verwendung gemäß Anspruch 5, wobei die Arterie oder Vene durch Stenose verstopft ist.

8. Verwendung gemäß Anspruch 7, wobei die Stenose eine Passage eines unzureichenden Volumens an Blut vor der Behandlung erlaubt.

9. Verwendung gemäß einem der vorangehenden Ansprüche, wobei das Subjekt an Koronarobstruktion leidet.

10. Verwendung gemäß einem der vorangehenden Ansprüche, wobei das Medikament durch einen Katheter verabreicht wird.

11. Verwendung gemäß einem der vorangehenden Ansprüche, wobei das Medikament direkt in die Wand der Arterie oder Vene verabreicht wird.

12. Verwendung gemäß einem der vorangehenden Ansprüche, wobei das Medikament eine Pankreas-Elastase umfasst.

13. Verwendung gemäß einem der vorangehenden Ansprüche, wobei das Medikament keine Kollagenase umfasst.

14. Verwendung gemäß einem der vorangehenden Ansprüche, wobei das Medikament eine Vergrößerung des Durchmessers der Arterie oder Vene durch Proteolyse des Elastins in der Wand der Arterie oder Vene von außen hinein verursacht.

## Revendications

1. Utilisation d'une élastase pour la fabrication d'un médicament pour une administration locale dans la paroi d'une artère ou veine pour élargir le diamètre de l'artère ou veine d'un sujet humain qui le nécessite.

2. Utilisation selon la revendication 1, dans laquelle l'artère ou veine est reliée à une greffe artérioveineuse pour hémodialyse chez le sujet humain.

3. Utilisation selon la revendication 1, dans laquelle le médicament est destiné à une administration locale dans la paroi d'une veine pour une utilisation dans l'hémodialyse.

4. Utilisation selon la revendication 3, dans laquelle le médicament est destiné à une administration dans la paroi de la veine pour une utilisation dans la formation d'une greffe artérioveineuse.

5. Utilisation selon l'une quelconque des revendications précédentes, dans laquelle l'artère ou veine est obstruée.

6. Utilisation selon la revendication 5, dans laquelle l'artère ou veine est obstruée par hyperplasie de l'intima.

7. Utilisation selon la revendication 5, dans laquelle l'artère ou veine est obstruée par sténose.

8. Utilisation selon la revendication 7, dans laquelle la sténose permet le passage d'un volume insuffisant de sang avant le traitement.

9. Utilisation selon l'une quelconque des revendications précédentes, dans laquelle le sujet souffre d'une obstruction eoronarienne.

10. Utilisation selon l'une quelconque des revendications précédentes, dans laquelle le médicament est administré par un cathéter.

11. Utilisation selon l'une quelconque des revendications précédentes, dans laquelle le médicament est administré directement dans la paroi de l'artère ou veine.

12. Utilisation selon l'une quelconque des revendications précédentes, dans laquelle le médicament comprend une élastase pancréatique.

13. Utilisation selon l'une quelconque des revendications précédentes, dans laquelle le médicament ne comprend pas de collagénase.

14. Utilisation selon l'une quelconque des revendications précédentes, dans laquelle le médicament entraîne l'élargissement du diamètre de l'artère ou veine par protéolyse d'élastine dans la paroi de l'artère ou veine depuis l'extérieur.
